(19) Europäisches Patentamt
European Patent Office
Office européen des brevets

(11) Numéro de publication: **0 211 746**
**B1**

(12) # FASCICULE DE BREVET EUROPEEN

(45) Date de publication du fascicule du brevet: **23.05.90**

(51) Int. Cl.$^5$: **C 07 D 241/04,** A 61 K 31/495

(21) Numéro de dépôt: **86401644.9**

(22) Date de dépôt: **23.07.86**

(54) **Dérivés de phényl-pipérazine, procédé de préparation et utilisation en thérapeutique.**

(30) Priorité: **31.07.85 FR 8511684**

(43) Date de publication de la demande:
**25.02.87 Bulletin 87/09**

(45) Mention de la délivrance du brevet:
**23.05.90 Bulletin 90/21**

(84) Etats contractants désignés:
**AT BE CH DE FR GB IT LI LU NL SE**

(56) Documents cités:
**FR-A-2 351 108**
**FR-M- 8 447**

(73) Titulaire: **LABORATOIRE L. LAFON Société anonyme dite:**
**1 rue Georges Médéric**
**F-94701 Maisons-Alfort (FR)**

(72) Inventeur: **Lafon, Louis**
**5, rue de l'Alboni**
**F-75016 Paris (FR)**

(74) Mandataire: **Clisci, Serge et al**
**S.A. FEDIT-LORIOT CONSEILS EN PROPRIETE INDUSTRIELLE 38, avenue Hoche**
**F-75008 Paris (FR)**

EP 0 211 746 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention a trait à de nouveaux produits industriels, à savoir des dérivés de phényl-pipérazine de formule I ci-après et leurs sels d'addition. L'invention concerne également leur procédé de préparation et leur utilisation en thérapeutique notamment en tant qu'agents antidépresseurs du système nerveux central (SNC).

On sait que l'on a déjà proposé dans le passé un certain nombre de dérivés de phényl-pipérazine. En particulier on a proposé dans FR—A—2 351 108, en tant qu'agents analgésiques, vasodilatateurs et antispasmodiques, des composés de formule

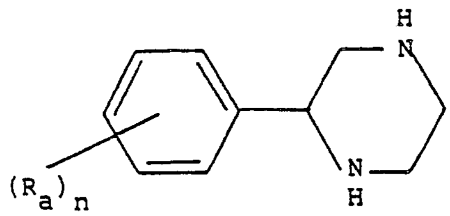

(Ia)

où $R_a$ représente un atome d'halogène, un groupe alkyle en $C_1$—$C_8$, alkoxy en $C_1$—$C_8$, nitro, cyano, benzyloxy, deux groupes R considérés ensemble pouvant former un reste méthylènedioxy, et n est un nombre entier ayant pour valeur 1, 2 ou 3.

On sait que l'on a également proposé dans FR—M—8477, en tant qu'agents dilateateurs coronariens pouvant posséder le cas échéant des effets sédatifs, des composés de formule

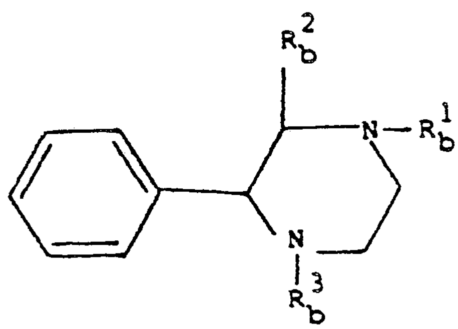

(Ib)

où $R_b^3$ représente (i) un groupe alkyle contenant moins de 5 atomes de carbone, (ii) un groupe phényle susceptible d'être substitué, ou (iii) un groupe phénylalkyl le susceptible d'être substitué sur le noyau phényle et dont le reste alkyle contient moins de 5 atomes de carbone; $R_b^1$ est un groupe alkyle contenant moins de 5 atomes de carbone ou un groupe phénylalkyle dans lequel le reste alkyle contient moins de 3 atomes de carbone; et, $R_b^2$ représente l'atome d'hydrogène ou un groupe alkyle contenant moins de 5 atomes de carbone.

On vient de trouver à présent que des composés de formule I ci-après, qui ne sont pas spécifiquement décrits ou sérieusement suggérés par l'art antérieur sus-visé, sont particulièrement intéressants en tant qu'agents antidépresseurs du SNC.

Les dérivés de phényl-pipérazine selon l'invention sont caractérisés en ce qu'ils sont choisis parmi l'ensemble constitué par

1) la 3-méthyl-2-phénylpipérazine,

2) la 1-isopropyl-3-phénylpipérazine,

3) la 1-éthyl-2-méthyl-3-phénylpipérazine,

4) la 1-isopropyl-2-méthyl-3-phénylpipérazine,

5) la 1,2,4-triméthyl-3-phénylpipérazine,

6) les (halogènophényl)-alkylpipérazines ayant dans leur molécule un atome d'halogène présent sur le cycle phényle et au moins un groupe alkyle présent sur le cycle pipérazinyle, et répondant à la formule générale

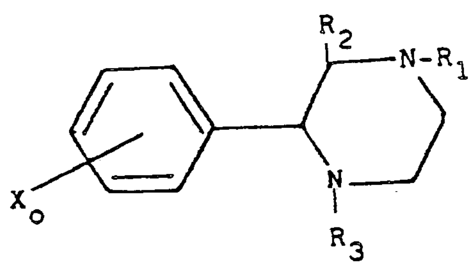

($I_o$)

dans laquelle $X_0$ est F, Cl ou Br, et $R_1$, $R_2$ et $R_3$ sont définis comme indiqué ci-dessus avec la condition

supplémentaire qu'un au moins desdits $R_1$, $R_2$ et $R_3$ est différent de H, et

7) leurs sels d'addition.

Conformément aux règles de nomenclatures de l'I.U.P.A.C., le cycle phényle des composés de formule I est indiqué comme étant fixé en position 2 ou 3 du cycle pipérazinyle en fonction de la localisation des autres substituants de celui-ci. C'est pourquoi dans ce qui suit, pour la nomenclature systématique des composés de formule I et de leurs intermédiaires de synthèse, on a attribué dans le cycle pipérazinyle

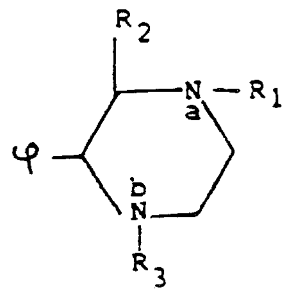

la position 1 à l'atome d'azote 'a' quand $R_1$ est différent de H ou, au contraire, à l'atome d'azote 'b' quand $R_1$ est H.

Par sels d'addition, on entend ici les sels d'addition d'acide obtenus par réaction d'une base libre de formule I ci-après avec un acide minéral ou organique, d'une part, et les sels d'ammonium, d'autre part. Parmi les acides utilisables pour salifier lesdites bases libres de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer les halogénures d'alkyle (notamment en $C_1$—$C_{10}$), d'aryle et d'aralkyle, en particulier les bromures, chlorures et iodures. D'une manière générale les sels d'additions d'acide sont préférés aux sels d'ammonium.

Un certain nombre de composés typiques selon l'invention a été consigné de façon nullement limitative dans le tableau I ci-après.

D'une manière générale, les composés selon l'invention peuvent être représentés par la formule

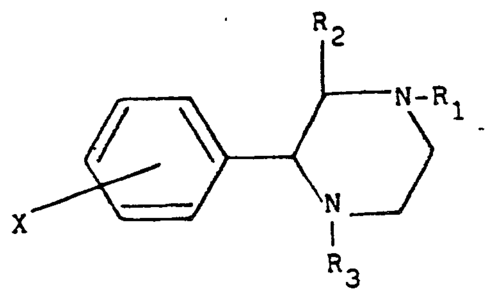

(I)

dans laquelle

(a) $X = R_1 = R_3 = H$, et $R_2 = CH_3$;

(b) $X = R_2 = R_3 = H$, et $R_1 = CH(CH_3)_2$;

(c) $X = R_3 = H$, $R_1 = CH_2CH_3$, et $R_2 = CH_3$;

(d) $X = R_3 = H$, $R_1 = CH(CH_3)_2$, et $R_2 = CH_3$;

(e) $X = H$, et $R_1 = R_2 = R_3 = CH_3$;

(f) X = F, Cl, ou Br, $R_1$ est H ou alkyle en $C_1$—$C_4$, $R_2$ est H ou alkyle en $C_1$—$C_4$, et $R_3$ est H ou alkyle en $C_1$—$C_4$, avec la condition supplémentaire dans la cadre de (f) que au moins un des $R_1$, $R_2$ et $R_3$ soit différent de H; et,

(g) leurs sels d'addition.

TABLEAU I

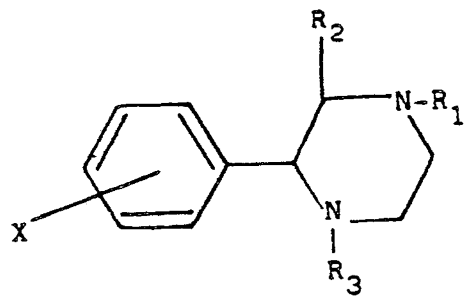

| Produit | No. de Code | X | $R_1$ | $R_2$ | $R_3$ | F (b) |
|---|---|---|---|---|---|---|
| Ex 1 (a) | CRL 41 223 | H | H | $CH_3$ | H | ≃ 260°C (c) |
| Ex 2 (a) | CRL 41 235 | H | $CH(CH_3)_2$ | H | H | 180—182°C (d) |
| Ex 3 (a) | CRL 41 270 | H | $CH_2CH_3$ | $CH_3$ | H | 198°C |
| Ex 4 (a) | CRL 41 271 | H | $CH(CH_3)_2$ | $CH_3$ | H | (e) |
| Ex 5 (a) | CRL 41 272 | H | $CH_3$ | $CH_3$ | $CH_3$ | 190°C |
| Ex 6 (a) | CRL 41 202 | 4-Cl | $CH_3$ | H | H | 200°C (c) |
| Ex 7 (a) | CRL 41 228 | 3-Cl | $CH_2CH_3$ | H | H | 170°C |
| Ex 8 (a) | CRL 41 234 | 2-F | $CH_2CH_3$ | H | H | 210°C (c) |
| Ex 9 (a) | CRL 41 238 | 2-Cl | $CH_2CH_3$ | H | H | 215°C (c) |
| Ex 10 (a) | CRL 41 239 | 4-Cl | $CH_2CH_3$ | H | H | 208°C (c) |
| Ex 11 (a) | — | 2-Br | $CH_2CH_3$ | H | H | 221°C |

Notes
(a) dichlorhydrate
(b) point de fusion instantanée
(c) avec décomposition
(d) produit hygroscopique
(e) produit huileux

De façon avantageuse le groupe $R_1$ représentera un groupe alkyle en $C_1$—$C_4$; $R_1$ pourra également représenter l'atome d'hydrogène lorsque le groupe $R_2$ sera un groupe alkyle en $C_1$—$C_2$.

Parmi les composés préférés selon l'invention eu égard à leurs effets sur le SNC, et en particulier leurs propriétés antidépressives, on peut notamment citer

a) les composés de formule I où $R_1$ est H ou alkyle en $C_1$—$C_4$, $R_2$ est alkyle en $C_1$—$C_2$, $R_3$ est H ou alkyle en $C_1$—$C_4$, et X est H;

b) les composés de formule I où $R_1$ est alkyle en $C_1$—$C_4$, $R_3$ est H ou aklyle en $C_1$—$C_2$, $R_3$ est H ou alkyle en $C_1$—$C_4$, et X est un atome d'halogène (et mieux 2-Cl, 2-Br ou 2-F).

Parmi ceux-ci, les composés les plua intéresants sur le plan thérapeutique sont les 3-méthyl-2-phényl-pipérazine, 1-éthyl-3-(2-chlorophényl)-pipérazine, 1-éthyl-3-(2-fluorophényl)-pipérazine, et leurs sels d'addition, notamment les dichlorohydrates.

Les composés de formule I peuvent être préparés selon une méthode connue en soi par application de

mécanismes réactionnels classiques. Le procédé de préparation que l'on préconise ici consiste en ce que

A) on fair réagir une 1-phényl-1,2-alcanedione de formule

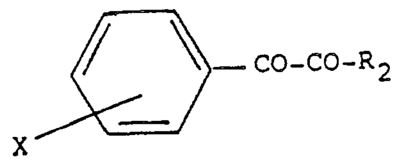

(II)

où X et $R_2$ sont définis comme ci-dessus, avec l'éthylènediamine

$$H_2NCH_2CH_2NH_2 \qquad (III)$$

pour obtenir une 2-phényl-dihydropyrazine de formule

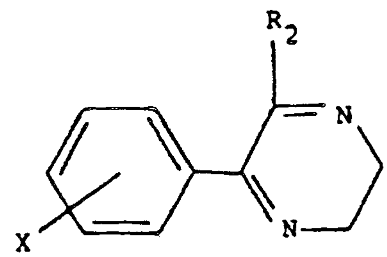

(IV)

où X et $R_2$ sont définis comme ci-dessus;

B) on soumet le composé de formule IV ainsi obtenu à une réaction de réduction au moyen d'un agent réducteur, notamment choisi parmi l'ensemble comprenant $LiAlH_4$ et $NaBH_4$ pour obtenir une 2-phenyl-pipérazine de formule I où $R_1 = R_3 = H$; et

C) si nécessaire, on soumet le composé de formule I où $R_1 = R_3 = H$ ainsi obtenu à une réaction d'alkylation pour introduire le groupe $R_1$ = alkyle en $C_1$—$C_4$ ou les groupes $R_1$ et $R_3$ représentant chacun un groupe alkyle en $C_1$—$C_4$.

La réaction de cyclisation du stade A est avantageusement mise en oeuvre en faisant intervenir un excès d'éthylènediamine par rapport aux conditions stoechiométriques (de préférence avec un rapport molaire III:II compris entre 1,1 et 1,5), pendant au moins 10 minutes à une température de 15—25°C. Comme solvant convenant pour la cyclisation du stade A on peut notamment citer les alcools inférieurs tels que $CH_3OH$ et $C_2H_5OH$.

La réaction de réduction du stade B est effectuée au moyen d'un borohydrure ou d'un aluminohydrure, à savoir ici $LiAlH_4$, $NaBH_4$ ou une substance similaire telle que $KBH_4$. Cette réaction est effectuée à une température de 0 à 25°C pendant au moins 10 minutes. En pratique la réaction de réduction avec l'aluminohydrure est réalisée à basse température (0 à 5°C) pendant 10 à 30 minutes, et celle avec le borohydrure à basse température (0 à 5°C) pendant 15 à 60 minutes puis pendant au moins 3 h à la température ambiante (15—20°C).

Au stade C la N-alkylation est effectuée selon une méthode classique pour obtenir, soit un dérivé de formule I N-monoalkylé ($R_1$ = alkyle en $C_1$—$C_4$; $R_3$ = H), soit un dérivé de formule I N,N'-dialkylé ($R_1$ = alkyle en $C_1$—$C_4$; $R_2$ = alkyle en $C_1$—$C_4$).

La N-monoalkylation est avantageusement réalisée par réaction d'un composé de formule I où $R_1 = R_3 = H$ avec un réactif choisi parmi l'ensemble comprenant

(i) les halogénures d'alkyle de formule

$$R_1' \text{ Hal} \qquad (V)$$

où $R_1'$ est un groupe alkyle en $C_1$—$C_4$ et Hal est un atome d'halogène, en milieu alcalin (présence d'ions $Na^+$ ou $K^+$), et

(ii) les esters d'acide carboxylique de formule

$$R_1''\text{—COO—Alk} \qquad (VI)$$

où Alk est un groupe alkyle en $C_1$—$C_3$ (notamment $CH_3$ et, de préférence, $C_2H_5$) et $R_1''$ est un groupe alkyle en $C_1$—$C_3$, en présence de $AlLiH_4$ dans un solvant inerte tel que le tétrahydrofuranne.

Au cours de la réaction de N-alkylation au moyen d'un halogénure d'alkyle $R_1'$ Hal (où Hal est F, Cl, Br ou I), on a constaté que les iodures tels que $ICH_3$ favorisaient l'alkylation de l'atome d'azote "a".

Dans la réaction de N-alkylation au moyen d'un ester de formule VI en présence du composé réducteur

$AlLiH_4$, c'est le reste acyle $R_1''CO$ qui intervient. Ainsi quand $R_1''$ est $CH_3$, le reste $CH_3CO$ conduit au groupe $R_1 = C_2H_5$, et quand $R_1''$ est $C_2H_5$, le reste $CH_3CH_2CO$ conduit au groupe ramifié $R_1 = CH(CH_3)_2$ selon le mécanisme réactionnel décrit par J. M. Khanna et al., Synthesis, septembre 1975, pages 607—608.

La N-monoalkylation susvisée concerne essentiellement l'atome d'azote "a". La N'-monoalkylation sur l'atome d'azote "b" peut être réalisée selon des modalités opératoires classiques, par exemple (i) en faisant appel à un catalyseur favorisant l'alkylation de l'atome d'azote "b" de préférence à l'atome d'azote "a", soit encore (ii) par réaction d'une 1-phényl-1,2-alcanedione de formule II avec une éthylènediamine de formule

$$H_2NCH_2CH_2NHR_3' \qquad \text{(III bis)}$$

où $R_3'$ est un groupe alkyle en $C_1$—$C_4$, puis isolation, du milieu réactonnel, du dérivé cyclisé résultant de formule I (où $R_3$ = alkyle en $C_1$—$C_4$ et $R_1$ = H) et de son isomère (composé de formule I où $R_3$ = H et $R_1 = R_3'$).

La méthode générale pour réaliser la N,N'-dialkylation consiste à soumettre un composé N-monoalkylé à une réaction de N'-alkylation selon une méthode connue. En variante on peut réaliser directement la N,N'-dialkylation, au moyen d'un formiate d'alkyle de formule VI (où $R_1''$ est H) en présence de $AlLiH_4$, pour obtenir un composé de formule I où $R_1 = R_3 = CH_3$.

Les composés selon l'invention agissent tous sur le SNC. Ils présentent la propriété commune d'intervenir dans l'organisme en tant qu'agents antidépreseurs. Dans leur profil neuropsychopharmacologique, à côté de leurs propriétés antidépressives, ils présentent également des effets stimulants et/ou sédatifs selon les doses administrées. Des produits de formule $I_o$, tels que le CRL 41 238 (produit de l'exemple 9), présentent en outre des effets immunomodulateurs bénéfiques.

Selon l'invention on préconise une composition thérapeutique qui est caractérisée en ce qu'elle renferme, en association avec un excipient physiologiquement acceptable, au moins un dérivé de phénylpipérazine comme défini ci-dessus ou l'un de ses sels d'addition non-toxiques, en tant qu'ingredient actif.

Bien entendu, dans une telle composition l'ingrédient actif, qui est choisi parmi l'ensemble comprenant lesdits dérivés de formule I, leurs sels d'addition non-toxiques et leurs mélanges intervient en quantité pharmaceutiquement efficace.

On préconise l'utilisation d'une substance appartenant à la famille des dérivés de phényl-pipérazine de formule I tels que définis ci-dessus, de leurs sels d'addition non-toxiques et de leurs mélanges pour l'obtention d'un médicament antidépresseur du SNC destiné à une utilisation thérapeutique vis-à-vis des dépressions et des étants dépressifs.

On préconise également l'utilisation de la 1-éthyl-3-(2-chlorophényl)-pipérazine, de ses sels d'addition non-toxiques, de leurs analogues de formule $I_o$ et de leurs mélanges pour l'obtention d'un médicament immuno-modulateur destiné à une utilisation immunologique.

D'autres avantages et caractéristiques de l'invention seront mieux compris à la lecture qui va suivre, d'une part, d'exemples de préparation, et, d'autre part, de résultats d'essais pharmacologiques, l'ensemble de ces éléments n'étant nullement limitatif mais donné à titre d'illustration.

Preparation I

Obtention du dichlorhydrate de 3-méthyl-2-phénylpipérazine

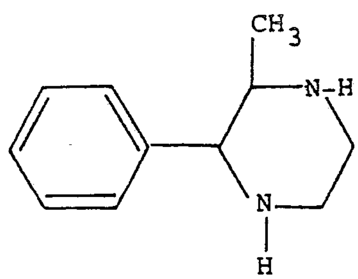

, 2 HCl

(Exemple 1; No. de Code: CRL 41 223).

On laisse en contact pendant 0,5 h une solution de 5 g (0,0388 mole) de 1-phényl-1,2-propanedione et de 2,5 g (0,0417 mole) d'éthylènediamine dans 300 ml de méthanol. On refroidit par un bain de glace, ajoute 5 g (0,132 mole) de $NaBH_4$ et laisse en contact pendant une nuit. On évapore à sec, reprend le résidu d'évaporation à l'eau, extrait au chloroforme, lave la solution chloroformique à l'eau et la sèche sur $MgSO_4$, filtre pour écarter $MgSO_4$ puis évapore à sec. On reprend le résidu d'évaporation par le méthanol, acidifie au moyen d'éthanol chlorhydrique ($C_2H_5OH$ contenant HCL 3N), évapore à sec, reprend le résidu

d'évaporation avec $CH_3COCH_3$ et filtre les cristaux formés. Par recristallisation du méthanol on obtient 3 g (rendement: 36%) de CRL 41 223. $F_{inst.} \approx 260°C$ (décomposition).

Analyse { % $Cl^-$ mesuré: 27,99%
% $Cl^-$ théorique: 28,51%

Preparation II
Obtention du dichlorhydrate de 1-éthyl-2-méthyl-3-phényl-pipérazine
(Exemple 3; No. de Code: CRL 41 270)

On coule une solution de 0,169 mole de 3-méthyl-2-phényl-pipérazine dans 250 ml de tétrahydrofuranne (THF) anhydre, dans une suspension de 25 g de $AlLiH_4$ dans 1000 ml de THF anhydre. On attend 0,25 h après la fin de l'introduction puis coule lentement 83 ml de $CH_3CO_2C_2H_5$. On attend 0,25 h après la fin de l'introduction puis coule avec précaution NaOH 3N. On filtre, évapore à sec le filtrat, reprend le résidu d'évaporation à l'eau, extrait au chloroforme, lave la phase chloroformique à l'eau et la sèche sur $MgSO_4$. On filtre (pour écarter $MgSO_4$), évapore à sec, reprend le résidu d'évaporation avec $CH_3OH$, acidifie avec de l'éthanol chlorhydrique et place le mélange résultant au réfrigérateur. On filtre (pour écarter le dichlorhydrate de 3-méthyl-2-phényl-pipérazine formé à partir de la base libre qui n'a pas réagi), évapore à sec le filtrat; par recristallisation du résidu d'évaporation du mélange diéthyléther-éthanol (1:1) v/v, on obtient 12 g (rendement: 25,6%) de CRL 41 270. $F_{inst.} = 198°C$.

Preparation III
Obtention du dichlorhydrate de 1-isopropyl-2-méthyl-3-phényl-pipérazine
(Exemple 4; No. de Code: CRL 41 271)

On dissout 42 g (0,169 mole) de dichlorhydrate de 3-méthyl-2-phényl-pipérazine (CRL 41 223, obtenu selon le procédé de la préparation I) dans $H_2O$, neutralise avec NaOH, extrait avec $CHCl_3$, lave la phase chloroformique à l'eau et sèche sur $MgSO_4$. On filtre, évapore à sec le filtrat puis reprend le résidu d'évaporation avec THF anhydre.

La solution résultante (qui renferme la base libre du CRL 41 223) est coulée dans une suspension de 25 g de $AlLiH_4$ dans 1000 ml de THF. On attend 0,25 h après fin d'introduction. On coule lentement 98 ml de $CH_3CH_2CO_2C_2H_5$ et attend 0,25 h après fin d'introduction. On coule ensuite avec précaution NaOH 3N, filtre, évapore à sec le filtrat, reprend le résidu d'évaporation avec $CH_3OH$, acidifie avec de l'éthanol chlorhydrique et place au réfrigérateur. On filtre le précipité de CRL 41 223 (aui s'est formé à partir de la base libre qui n'a pas réagi), évapore à sec le filtrat. Par recristallisation du résidu d'évaporation du mélange diéthyléther-éthanol (1:1 v/v, on obtient 7,8 g (rendement: 16%) de CRL 41 271 se présentant sous la forme d'une huile.

Preparation IV
Obtention du dichlorhydrate de 1-éthyl-3-(2-chlorophényl)-pipérazine
(Exemple 9; No. de Code: CRL 41 238)

On dissout 18,86 g (0.070 mole de dichlorhydrate de 2-(2-chlorophényl)-pipérazine ($F_{inst.} = 230°C$; produit décrit dans FR—A—2 351 108, voir page 6 ligne 1 dudit document français) dans $H_2O$, neutralise avec NaOH, extrait avec $CHCl_3$, lave la solution chloroformique à l'eau et la sèche par $MgSO_4$, on filtre, évapore à sec le filtrat, reprend la résidu d'évaporation par 200 ml de THF anhydre. On coule cette solution dans une suspension de 10 g de $AlLiH_4$ dans 1000 ml de THF anhydre. On attend 10 minutes puis coule lentement 20 ml de $CH_3CO_2C_2H_5$. On laisse le milieu réactionnel évoluer pendant 0,5 h et ajoute NaOH 3N. On filtre, évapore à sec le filtrat, reprend le résidu d'évaporation avec $H_2O$, extrait avec $CHCl_3$, lave la solution chloroformique avec $H_2O$ et séche sur $MgSO_4$. On filtre, évapore à sec le filtrat, reprend le résidu d'évaporation avec $CH_3OH$, acidifie avec de l'éthanol chlorhydrique. Après refroidissement au réfrigérateur on écarte par filtration le précipité formé [qui contient le dichlorhydrate de 2-(2-chlorophényl)-pipérazine], évapore à sec le filtrat; par recristallisation du résidu d'évaporation du mélange acétone-éthanol (1:1) v/v on obtient 7,2 g (rendement: 34,6%) de CRL 41 238. $F_{inst.} = 215°C$ (décomposition).

Preparation V
Obtention du dichlorhydrate de 1-méthyl-3-(4-chlorophényl)-pipérazine
(Exemple 6; No. de Code: CRL 41 202)

On chauffe à reflux pendant 6 h un mélange de 10 g (0,051 mole) de 2-(4-chlorophényl)-pipérazine, 10,80 g (0,102 mole) de $Na_2CO_3$, 7,24 g (0,051 mole) de $ICH_3$ et 100 ml d'eau. On refroidit, extrait avec $CHCl_3$, lave la phase chloroformique avec de l'eau et la sèche sur $MgSO_4$. On filtre, évapore à sec le filtrat, reprend le résidu d'évaporation avec $CH_3OH$, acidifie avec de l'éthanol chlorhydrique. On évapore à sec; par recristallisation du résidu d'évaporation du mélange méthanolacétate d'éthyle (1:1) v/v, on obtient 3 g (rendement: 20,8%) de CRL 41 202. $F_{inst.} = 200°C$ (décomposition).

Preparation VI

Obtention du dichlorhydrate de 1,2,4-triméthyl-3-phényl-pipérazine

(Exemple 5; No. de Code: CRL 41 272)

On coule une solution de 0,169 mole de 3-méthyl-2-phényl-pipérazine dans le THF anhydre, dans une suspension de 25 g de $AlLiH_4$ dans 1000 ml de THF. On attend 0,25 h après fin d'introduction, puis coule lentement 67 ml de formiate d'éthyle. On attend 0,25 h après fin d'introduction puis coule avec précaution NaOH 3N. On filtre, évapore à sec le filtrat, reprend le résidu d'évaporation avec $H_2O$, extrait avec $CHCl_3$, lave la solution chloroformique avec $H_2O$ et la sèche sur $MgSO_4$. On filtre, évapore à sec le fltrat, reprend le résidu d'évaporation avec $CH_3OH$, acidifie avec de l'éthanol chlorhydrique. On place le mélange résultant au réfrigerateur, filtre le précipité de CRL 41 223 (qui s'est formé à partir de la base libre correspondante qui n'a pas réagi), évapore à sec le filtrat, puis par recristallisation du résidu d'évaporation du mélange diéthyléther-éthanol (1:1) v/v on obtient 13 g (rendement: 28%) de CRL 41 272. $F_{inst.}$ = 190°C.

Preparation VII

Obtention du chlorhydrate de 1-isopropyl-3-phényl-pipérazine

(Exemple 2; No. de Code CRL 41 235)

En procédant comme indiqué dans la préparation V ci-dessus, en remplaçant $ICH_3$ par $ICH(CH_3)_2$ on obtient le CRL 41 235 avec un rendement de 25%. F = 180—182°C (produit hygroscopique).

On a résumé ci-après des essais qui ont été entrepris avec les composés selon l'invention.

A. Essais Relatifs au CRL 41 238 (Produit de l'Exemple 9)

Dans l'étude neuropsychopharmacologique qui suit le CRL 41 238 en solution dans de l'eau distillée a été administré par voie intrapéritonéale sous un volume de 20 ml/kg chez la souris mâle et de 5 ml/kg chez le rat mâle.

Le pH de la solution injectée varie en fonction de la concentration du CRL 41 238 comme indiqué dans le tableau II ci-après.

TABLEAU II

pH du CRL 41 238
en fonction de la concentration

| Concentration | pH |
|---|---|
| 50 g/l | 2,0 |
| 26 g/l | 2,5 |
| 13 g/l | 3,0 |
| 6 g/l | 3,5 |
| 2 g/l | 4,0 |
| 0,8 g/l | 4,5 |
| 0,4 g/l | 5,0 |
| ≤0,05 g/l | 5,5 |

I. Toxicite

Chez la souris mâle la DL—O (dose maximale non mortelle) par voie intrapéritonéale est supérieure à 128 mg/kg et la DL—100 (dose minimale mortelle pour tous les animaux testés) est inférieure ou égale à 256 mg/kg.

II. Comportement Global et Reactivites

Des lots de trois animaux sont observés avant, puis 0,25 h, 0,50 h, 1 h, 2 h, 3 h et 24 h après l'administraton de CRL 41 238. On constate:

1) *chez la souris*
aux doses de 1 et 4 mg/kg:
pas de modification nette de comportement et des réactivités par rapport au lot témoin ne recevant que de l'eau,
une hypothermie à la dose de 4 mg/kg;

à la dose de 16 mg/kg:
une sédation durant de 0,5 h à 1 h,
une hypothermie,
une piloérection durant 1 h; et

à la dose de 64 mg/kg:
une sédation pendant 3 h,
une diminution de la fréquence respiratoire pendant 1 h,
une diminution de la réactivité au toucher pendant 0,25 h,
une hypothermie pendant 3 h, l'effet maximal (−3,2°C) étant atteint 0,5 h après administration;

2) *chez le rat*
aux doses de 0,5 mg/kg, 2 mg/kg et 8 mg/kg:
des comportements, réactivités, variations du diamètre pupillaire et de la température rectale sensiblement comparables à ceux du lot témoin ne recevant que de l'eau distillée; et

à la dose de 32 mg/kg:
une sédation et une diminution de la fréquence rspiratoire pendant 3 h,
une diminution de la réactivité au toucher et du tonus musculaire pendant 1 h,
une mydriase modérée pendant 1 h,.

III. Interaction avec l'Apomorphine
1) *chez la souris*
Des lots de 6 souris reçoivent le CRL 41 238 0,5 h avant l'injection sous-cutanée de 1 ou 16 mg/kg d'apomorphine. On observe que, à la forte dose utilisée (64 mg/kg), le CRL 41 238 s'oppose à l'hypothermie induite par 16 mg/kg d'apomorphine, et que les comportements de verticalisation et les stéréotypies induits par 1 mg/kg et 16 mg/kg d'apomorphine ne sont pratiquement pas modifiés par le CRL 41 238 aux doses utilisées.

2) *Chez le rat*
Le CRL 41 238 est administré à des lots de 6 rats 0,5 h avant l'injection sous-cutanée de 0,5 mg/kg d'apomorphine. On Observe que le CRL 41 238 ne modifie pas les stéréotypies induites par l'apomorphine.

IV. Interaction avec l'Amphetamine
L'amphétamine (2 mg/kg) est injectée par voie intrapéritonéale à des lots de 6 rats, 30 minutes après l'administration de CRL 41 238. On constate que, à la plus forte dose utilisée (32 mg/kg), le CRL 41 238 ne modifie pas les stéréotypies amphétaminiques.

V. Interaction avec la Reserpine
Quatre heures après l'injection intrapéritonéale de 2,5 mg/kg de réserpine, des lots de 6 souris reçoivent le CRL 41 238.
On note que le CRL 41 238 ne s'oppose pas à l'hypothermie et au ptôsis induits par la réserpine.

VI. Interaction avec l'Oxotremorine
Le CRL 41 238 est administré à des lots de 6 souris 0,5 h avant l'injection intrapéritonéale de 0,5 mg/kg d'oxotrémorine.

1) *Action sur la température*
On constate que, à la plus forte dose utilisée (64 mg/kg), le CRL 41 238 s'oppose à l'action hypothermisante de l'oxotrémorine, alors que ledit CRL 41 238 dès la dose de 4 mg/kg et surtout aux doses de 16 et 32 mg/kg, est hypothermisant quand il est administré seul.

2) *Action sur les tremblements*
On constate que, à la plus forte dose utilisée (64 mg/kg), le CRL 41 238 diminue l'intensité des tremblements induits par l'oxotrémorine.

3) *Action sur les symptômes cholinergiques périphériques*
On observe que le CRL 41 238 ne modifie pas les signes de stimulation cholinergique périphérique induits par l'oxotrémorine.

VII. Action Sur le Test des Quatre Plaques, la Traction et l'Electochoc

Le test est pratiqué sur des lots de 10 souris, 30 minutes après l'administration de CRL 41 238.

On constate que le CRL 41 238 ne modifie pas le nombre de passages punis, ne provoque pas d'incapacité motrice majeure, n'aggrave pas les effets convulsivants et létaux de l'électrochoc.

VIII. Action Sur la Motilite Spontanee

0,5 h après avoir reçu le CRL 41 238, les souris (6 par dose, 12 témoins) sont placées en actimètre où leur motilité est enregistrée pendant 30 minutes.

On observe que, dès la dose de 1 mg/kg et surtout aux doses de 4 mg/kg et 64 mg/mg, le CRL 41 238 diminue l'activité motrice spontanée de la souris.

IX. Action Sur l'Agressivite Intergroupes

Après avoir séjourné pendant 3 semaines dans chacune des moitiés d'une cage séparées par une cloison opaque, des groupes de 3 souris reçoivent le CRL 41 238. Une demi-heure plus tard, les deux groupes d'une même cage sont réunis par retrait de la cloison et on note le nombre de combats qui surviennent en 10 minutes.

On constate que le CRL 41 238 diminue faiblement le nombre de combats. Il convient de remarquer que les résultats ne sont pas significatifs dès lors que les témoins étaient de façon anormale non combatifs.

X. Action Vis-A-Vis de Quelques Comportements Perturbes Par Divers Agents

1) *Motilité réduite par habitation à l'enceinte*

Après 18 heures de séjour dans les actimètres, les souris (6 par dose, 12 témoins) reçoivent le CRL 41 238. Elles sont aussitôt replacées dans leurs enceintes respectives et, une demi-heure plus tard, on enregistre leur motilité pendant 30 minutes.

On observe que le CRL 41 238 n'entraîne pas de reprise nette de l'activité motrice chez la souris habituée à son enceinte.

2) *Motilité réduite par agression hypoxique*

Une demi-heure après avoir reçu le CRL 41 238, les souris (10 par dose, 20 témoins) sont soumises à une anoxie hypobare aiguë [dépression de 600 mmHg (i.e. environ $8 \times 10^4$ Pa) en 90 secondes; détente de 45 secondes], puis elles sont placées en actimètre où leur motilité est enregistrée pendant 10 minutes.

On observe que le CRL 41 238 n'entraîne pas d'amélioration nette de la récupération motrice chez la souris dont la motilité a été déprimée à la suite d'un séjour bref dans une enceinte à pression téduite.

3) *Anoxie asphyxique*

Des lots de 10 souris reçoivent le CRL 41 238 une demi-heure avant l'administration intrapéritonéale de 32 mg/kg de triiodoéthylate de gallamine (agent curarisant de référence).

On observe que le CRL 41 238 ne modifie pratiquement pas le délai d'apparition des convulsions et de la mort consécutives à une anoxie asphyxique provoquée par un curarisant.

XI. Interaction Avec le Barbital

Une demi-heure après l'administration de CRL 41 238, des lots de 10 souris reçoivent une injection intrapéritonéale de barbital (220 mg/kg).

On constate que le CRL 41 238 ne modifie pas la durée du sommeil barbiturique.

XII. Action Sur le "Desespoir Comportemental"

Une demi-heure après reçu le CRL 41 238, des lots de 6 souris sont placés dans un bécher rempli d'eau sur une hauteur de 6 cm. On note la durée totale d'immobilité entre la 2ème et a 6ème minutes suivant l'immersion.

On observe que, dès la dose de 4 mg/kg et surtout aux doses de 8 mg/kg, 16 mg/kg, 32 mg/kg et 64 mg/kg, le CRL 41 238 diminue la durée de l'immobilité de la souris placée en immersion forcée.

XIII. Conclusions

Il résulte de l'ensemble des essais neuropsychopharmacologiques ci-dessus que le CRL 41 238 présente des effets.

*antidépresseurs*: antagonisme modéré des hypothermies induites par l'apomorphine, la réserpine ou l'oxotrémorine à doses élevées chez la souris, et, diminution nette de la durée de l'immobilité du "désespoir" dès doses les plus faibles;

*sédatifs*: sédation chez la souris et le rat avec diminution des réactivités, hypothermie chez la souris, et, diminution de l'activité motrice spontanée che la souris.

XIV. Complement (Etude Immunologique)

Le CRL 41 238 présente, à la dose de 100 mg/kg per os, une activité sur l'immunité cellulaire et humorale selon le test dit des cellules formant des plages de lyse décrit par A. J. Cunningham et al. ("Further improvements in the plaque technique for detecting single antibody forming cells"), Immunology

*14*, pages 599—601, (1968), et selon la mesure de l'intensité de l'hypersensibilité retardée aux globules rouges de mouton décrite par T. E. Miller et al. ("Immunopotentiation with BCG II modulation of the response to sheep blood cells"), Journal of the National Cancer Institute *51* (No. 5), pages 1669—1676, (1973). Il résulte des essais correspondants que le CRL 41 238 se comporte comme un agent immunomodulateur.

En clinique le CRL 41 238 s'est avéré être un bon agent antidépresseur, en particulier dans le traitement des patients atteints (i) de dépressions psychotiques de type mélancolique, et (ii) d'états dépressifs de type névrotique. Chez l'adulte la posologie préconisée est de 45 à 90 mg de CRL 41 238 per os par jour, notamment à raison de 3 à 6 gélules quotidiennes renfermant chacune 15 mg de principe actif.

B. Essais Relatifs Au CRL 41 223 (Produit de l'Exemple 1)

L'etude neuropsychopharmacologique du CRL 41 223 a été réalisée comme indiqué ci-dessus pour le CRL 41 238, avec la différence que l'administration par voie intrapéritonéale chez la souris mâle (sous un volume de 20 ml/kg) et le rat mâle (sous un volume de 5 ml/kg) est intervenue après avoir mis le CRL 41 223 en suspension dans une solution aqueuse de gomme arabique pour les concentrations supérieures à 25 g/l ou en solution dans de l'eau distillée pour les concentrations inférieures ou égales à 25 g/l (pH 4,5).

a) Toxicite

Par voie intrapéritonéale chez la souris mâle le CRL 41 223 présente une DL—O supérieure à 256 mg/kg et une DL—100 inférieure ou égale à 512 mg/kg.

b) Comportement Global et Reactivites

1) *Chez la souris*

Le CRL 41 223 provoque par voie intrapéritonéale chez la souris, aux doses de

2 mg/kg:
une mydriase modérée pendant 3 h,

8 mg/kg:
une hypothermie qui est maximale (−1,4°C) 1 h après administration, et
une mydriase modérée pendant 3 h;

32 mg/kg:
une sédation débutant 0,25 h après administration,
une hypothermie qui est maximale (−3,3°C) 0,5 h après administration,
une mydriase modérée pendant 3 h,
une piloérection, et
une dyspnée;

128 mg/kg:
une sédation fugace (durant 0,25 h) pour les 2/3 des animaux puis une excitation 1 h après administration avec augmentation de la réaction de peur et de la réactivité au toucher (pendant 1 h),
une dyspnée,
une piloérection,
une hypothermie qui est maximale (−2,3°C) 0,50 h après administration, et
une mydriase pendant 3 h.

2) *Chez le rat*

Le CRL 41 223 provoque par voie intrapéritonéale chez le rat mâle, aux doses de

1 mg/kg:
une mydriase modérée pendant 3 h;

4 mg/kg:
une sédation pendant 2 h,
une piloérection, et
une mydriase importante pendant 3 h;

16 mg/kg:
une sédation pendant 2 h, avec diminution du tonus musculaire et de la réactivité au toucher,
une piloérection, et
une mydriase importante pendant 3 h;

64 mg/kg:

une sédation fugace 0,25 h après administration suivie d'une excitation pendant 3 h avec augmentation de la réactivité au toucher et au bruit,

une dyspnée,

une piloérection pendant 0,5 h, et

une mydriase modérée pendant 3 h.

c) Profil Neuropsychopharmacologique

Il résulte de l'ensemble des autres essais entrepris selon les protocoles donnés ci-dessus pour le CRL 41 228, que le CRL 41 223 présente dans son profil neuropsychopharmacologique:

*des effets antidépresseurs* objectivés par l'antagonisme des hypothermies induites par l'apomorphine, la réserpine et l'oxotrémorine, d'une part et la diminution de la durée de l'immobilité dite de "désespoir", d'autre part; et

*des effets biphasiques* qui se manifestent en fonction des doses:

*aux doses faibles, des effets sédatifs:*

diminution de l'activité motrice spontanée chez la souris,

hypothermie, et

*aux doses élevés, des effets excitants:*

antagonisme du sommeil barbiturique,

augmentation modérée de la réactivité au toucher,

potentialisation des stéréotypies induites par l'apomorphine et l'amphétamine,

augmentation modérée de l'activité motrice spontanée chez la souris, et, de la récupération motrice chez la souris dont l'activité a été déprimée par un séjour bref dans une enceinte à pression réduite; et enfin

des effets reflétant une *stimulation alpha-adrénergique périphérique* objectivés par l'antagonisme du ptôsis réserpinique, une mydriase, une piloérection, et, semble-t-il, l'antagonisme des tremblements provoqués par l'oxotrémorine.

d) Essais Complementaires

L'étude du CRL 41 223 a été entreprise sur le plan cardiovasculaire par administration en solution dans du sérum physiologique (concentration maximale utilisée 56 g/l; pH 4) par voie intraduodénale.

1) Un chien (poids: 13,9 kg) anesthésié au Nembutal® reçoit le CRL 41 223 par voie intraduodénale, aux doses successives de 0,1, 0,5, 1, 2,5, 5, 10 et 20 mg/kg. On mesure la pression artérielle, la fréquence cardiaque, le débit artériel fémoral, le débit artériel vertébral et les températures rectale et cutanée.

On constate que (i) le CRL 41 223 est légèrement hypertenseur à partir de la dose de 2,5 mg/kg par augmentation de la pression artérielle systolique, ne modifie pas la fréquence cardiaque, diminue le débit de l'artère fémorale, augmente le débit vertébral à partir de la dose de 20 mg/kg, et, provoque une augmentation modérée des températures rectale et cutanée.

2) Les effets de l'isoprénaline, testés chez le méme chien après la dose cumulée de 39,1 mg/kg I.D., ne sont pas modifiés. A 10 µg/kg d'isoprénaline, la pression artérielle diastolique passe de 132 mmHg (soit environ $1{,}75 \times 10^4$ Pa) à 24 mmHg (soit environ $3{,}2 \times 10^3$ Pa) avec le CRL 41 223, au lieu de 136 mmHg (soit environ $1{,}81 \times 10^4$ Pa) à 24 mmHg (soit environ $3{,}2 \times 10^3$ Pa) en témoin, et la fréquence cardiaque passe de 190 bat/min. à 260 bat/min. au lieu de 160 bat/min. à 270 bat/min. en valeur témoin.

3) L'hypertension induite par la noradrénaline chez le même chien est fortement augmentée par le CRL 41 223. A 2 µg/kg de noradrénaline, la pression artérielle systolique passe de 160 mmHg (soit environ $2{,}13 \times 10^4$ Pa) à 344 mmHg (soit environ $4{,}58 \times 10^4$ Pa) avec le CRL 41 223, au lieu de 152 mmHg (soit environ $2{,}02 \times 10^4$ Pa) à 280 mmHg (soit environ $3{,}73 \times 10^4$ Pa) en témoin.

e) Etudes Cliniques

En clinique le CRL 41 223 s'est avéré chez l'homme adulte étre un bon agent antidépresseur du SNC, particulièrement intéressant dans les cas d'insomnies à la dose quotidienne de 150 à 250 mg par voie orale. La posologie recommandée par voie orale est de 2 gélules par jour renfermant chacune 100 mg de CRL 41 223.

C. Essais Relatifs Aux Autres Produits

Les essais réalisés avec les autres produits selon l'invention sur le plan neuropsychopharmacologique montrent notamment que les CRL 41 325 (exemple 2), CRL 41 270 (exemple 3), CRL 41 271 (exemple 4), CRL 41 272 (exemple 5), CRL 41 202 (exemple 6), CRL 41 228 (exemple 7), CRL 41 234 (exemple 8) et CRL 41 239 (exemple 10) présentent tous des effets antidépresseurs (objectivés par l'antagonisme des hypothermies provoquées par l'apomorphine, la réserpine et l'oxotrémorine, d'une part, et la diminution de la durée d'immobilisation dite de désespoir, d'autre part) et des effets sédatifs (objectivés par l'hypothermie induite chez la souris, d'une part, et la diminution de l'activité motrice spontanée de la souris, d'autre part.

Il convient toutefois de noter quelques mécanismes d'action particuliers dans l'organisme pour les produits suivantes:

le CRL 41 270 (exemple 3) et le CRL 41 239 (exemple 10) provoquent de façon paradoxale une diminution de la durée du sommeil barbiturique chez la souris aux doses élevées;

le CRL 41 235 (exemple 2), le CRL 41 202 (exemple 6), le CRL 41 228 (exemple 7) et le CRL 41 239 (exemple 10) exercent des effets reflétant une stimulation alpha-adrénergique périphérique;

et

le CRL 41 234 (exemple 8) administré par voies orale et gastrique présente des effets antidépresseurs qui se manifestent principalement 0,5 h après administration, et, des effets sédatifs qui sont particulièrement bénéfiques.

Pour information, on a consigné dans le tableau III ci-après les résultats numériques d'essais relatifs aux produits des exemples 2—8 et 10 selon l'invention, en ce qui concerne la toxicité et les effets sédatifs (dans ce cas d'espèce, l'hypothermie induite par lesdits produits) chez la souris mâle par voie intrapéritonéale.

Une étude tératogène a été entreprise chez la lapine (Fauve de Bourgogne pesant environ 2900 g avant gestation; les femelles de la variété Fauve de Bourgogne ont en général une portée de 2 à 10 petits). Le premier jour de la gestation (i.e. le jour où le mâle est introduit dans la cage des femelles) les femelles sont réparties en lots de 10 animaux par dose et produits à tester et en un lot de 15 animaux pour contrôle. Les lapines reçoivent quotidiennement par gastrogavage O (lot contrôle), 5 mg/kg et 10 mg/kg des produits à tester, du 5ème au 19ème jour de la gestation. Une césarienne est effectuée le 28ème jour de la gestation afin de compter:

(i) le nombre de lapines présentant dans leur portée au moins une malformation foetale, et

(ii) le nombre total de foetus présentant une ou plusieurs malformations.

Les résultats de ces essais comparatifs mettent en évidence le fait que les produits selon l'invention, notamment le CRL 41 238, sont dépourvus des effets tératogènes néfastes à la différence des dérivés de phényl-pipérazine connus.

TABLEAU III

| Produit | No. de Code | Toxicité souris mâles i.p. (mg/kg) | Hypothermie souris mâle i.p. | | |
|---|---|---|---|---|---|
| | | | Dose (mg/kg) | Durée (h) | Valeur maximale |
| Ex. 2 | CRL 41 235 | DL—O > 128<br>DL—60 ≃ 250<br>DL—100 < 512 | 4<br>16<br>64 | 1<br>3<br>3 | −1,2°C à T + 0,5 h<br>−1,1°C à T + 0,75 h<br>−2,6°C à T + 1h |
| Ex. 3 | CRL 41 270 | DL—O > 128<br>DL—100 ≤ 256 | 64 | 3 | −2,5°C à T + 0,5 h |
| Ex. 4 | CRL 41 271 | DL—30 ≃ 60<br>DL—60 ≃ 125 | 16 | 1 | −0,8°C à T + 0,5 h |
| Ex. 5 | CRL 41 272 | DL—O > 128<br>DL—30 ≃ 250<br>DL—100 ≤ 512 | 16<br>64 | 0,5<br>3 | −1,3°C à T + 0,3 h<br>−3.4°C à T + 0,5 h |
| Ex. 6 | CRL 41 202 | DL—O > 256<br>DL—100 ≤ 256 | 32 | 3 | −1,2°C à T + 2 h |
| Ex. 7 | CRL 41 228 | DL—O > 64<br>DL—100 ≤ 256 | 32 | 3 | −1,2°C à T + 2 h |
| Ex. 8 | CRL 41 234 | DL—O > 128<br>DL—60 ≃ 250 | 16<br>64 | 2<br>2 | −3,1°C à T + 1 h<br>−3,0°C à T + 1 h |
| Ex. 10 | CRL 41 239 | DL—O > 128<br>DL—60 ≃ 250 | 16<br>64 | 2<br>3 | −0,9°C à T + 1 h<br>−3,0°C à T + 0,5 h |

**Revendications pour les Etats contractants: BE CH DE FR GB IT LI LU NL SE**

1. Dérivé appartenant à la famille des phényl-pipérazines substituées, caractérisé en ce qu'il est choisi parmi l'ensemble comprenant

1) la 3-méthyl-2-phénylpipérazine,
2) la 1-isopropyl-3-phénylpipérazine,
3) la 1-éthyl-2-méthyl-3-phénylpipérazine,
4) la 1-isopropyl-2-méthyl-3-phénylpipérazine,
5) la 1,2,4-triméthyl-3-phénylpipérazine,
6) les (halogènophényl)-alkylpipérazines ayant dans leur molécule un atome d'halogène présent sur le cycle phényle et au moins un groupe alkyle présent sur le cycle pipérazinyle, et répondant à la formule générale

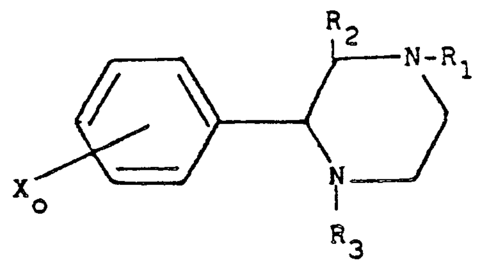

$(I_o)$

dans laquelle $X_0$ est F, Cl ou Br, et $R_1$, $R_2$, et $R_3$ sont définis comme indiqué ci-dessus avec la condition supplémentaire qu'un au moins desdits $R_1$, $R_2$, et $R_3$ est différent de H, et

7) leurs sels d'addition.

2. Dérive selon la revendication 1, caractérisé en ce qu'il est choisi parmi les (halogénophényl)-alkylpipérazines répondant à la formule

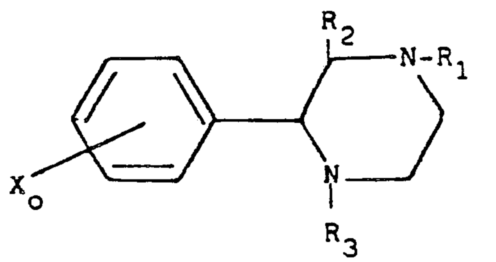

$(I_o)$

dans laquelle $X_o$ est F, Cl, ou Br et $R_1$, $R_2$, et $R_3$ sont définis comme indiqué ci-dessus, un au moins desdits $R_1$, $R_2$ et $R_3$ étant différent de H; et leurssels d'addition.

3. Dérivé selon la revendication 2, caractérisé en ce que $X_o$ est 2-F, 2-Cl ou 2-Br.

4. 1-Ethyl-3-(2-fluorophényl)-pipérazine et ses sels d'addition.

5. 3-Méthyl-2-phényl-pipérazine et ses sels d'addition.

6. 1-Ethyl-3-(2-chlorophényl)-pipérazine et ses sels d'addition.

7. 1,2,4-Triméthyl-3-phényl-pipérazine et ses sels d'addition.

8. Composition thérapeutique, caractérisée en ce qu'elle renferme, en association avec un excipient physiologiquement acceptable, au moins un dérivé de phényl-pipérazine selon la revendication 1 ou l'un de ses sels d'addition non-toxiques.

9. Utilisation d'un substance appartenant à la famille des dérivés de phényl-pipérazine selon la revendication 1 et de leurs sels d'addition non-toxiques, pour l'obtention d'un médicament antidépresseur destiné à une utilisation thérapeutique vis-à-vis des dépressions et des états dépressifs.

10. Procédé de préparation d'un dérivé appartenant à la famille des phényl-pipérazines selon la revendication 1, et choisi parmi l'ensemble constitué par les composés de formule

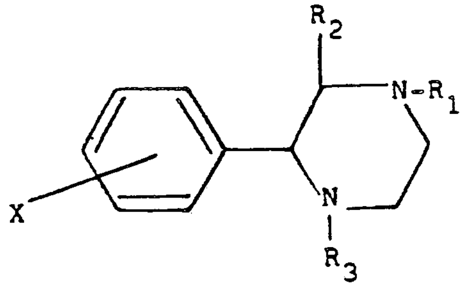

(I)

dans laquelle

(a) $X = R_1 = R_3 = H$, et $R_2 = CH_3$;
(b) $X = R_2 = R_3 = H$, et $R_1 = CH(CH_3)_2$;
(c) $X = R_3 = H$, $R_1 = CH_2CH_3$, et $R_2 = CH_3$;
(d) $X = R_3 = H$, $R_1 = CH(CH_3)_2$, et $R_2 = CH_3$;
(e) $X = H$, et $R_1 = R_2 = R_3 = CH_3$;
(f) X = F, Cl, ou Br, $R_1$ est H ou alkyle en $C_1$—$C_4$, $R_2$ est H ou alkyle en $C_1$—$C_4$, et $R_3$ est H ou alkyle en $C_1$—$C_4$, avec la condition supplémentaire dans la cadre de (f) que au moins un des $R_1$, $R_2$ et $R_3$ soit différent de H; et,
(g) leurs sels d'addition.

caractérisé en ce que

A) on fair réagir une 1-phényl-1,2-alcanedione de formule

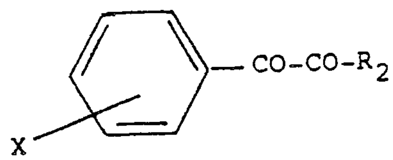

(II)

où X et $R_2$ sont définis comme ci-dessus, avec l'éthylènediamine

$$H_2NCH_2CH_2NH_2 \qquad (III)$$

pour obtenir une 2-phényl-dihydropyrazine de formule

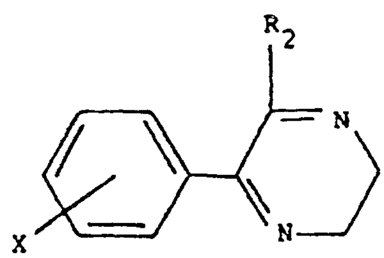

(IV)

où X et $R_2$ sont définis comme ci-dessus;

B) on soumet le composé de formule IV ainsi obtenu à une réaction de réduction au moyen d'un agent réducteur, notamment choisi parmi l'ensemble comprenant $LiAlH_4$ et $NaBH_4$ pour obtenir une 2-phenyl-pipérazine de formule I où $R_1 = R_3 = H$; et

C) si nécessaire, on soumet le composé de formule I où $R_1 = R_3 = H$ ainsi obtenu à une réaction d'alkylation pour introduire le groupe $R_1$ = alkyle en $C_1$—$C_4$ ou les groupes $R_1$ et $R_3$ représentant chacun un groupe alkyle en $C_1$—$C_4$.

**Revendication pour l'Etat contractant: AT**

Procédé de préparation d'un dérivé apparentant à la famille des phényl-pipérazines substituées et choisi parmi l'ensemble constitué par les composés de formule

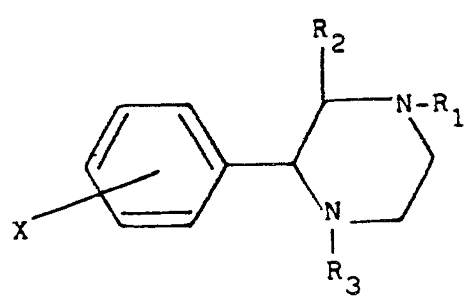

(I)

dans laquelle

(a) $X = R_1 = R_3 = H$, et $R_2 = CH_3$;
(b) $X = R_2 = R_3 = H$, et $R_1 = CH(CH_3)_2$;
(c) $X = R_3 = H$, $R_1 = CH_2CH_3$, et $R_2 = CH_3$;
(d) $X = R_3 = H$, $R_1 = CH(CH_3)_2$, et $R_2 = CH_3$;
(e) $X = H$, et $R_1 = R_2 = R_3 = CH_3$;
(f) X = F, Cl, ou Br, $R_1$ est H ou alkyle en $C_1$—$C_4$, $R_2$ est H ou alkyle en $C_1$—$C_4$, et $R_3$ est H ou alkyle en

$C_1$—$C_4$, avec la condition supplémentaire dans la cadre de (f) que au moins un des $R_1$, $R_2$ et $R_3$ soit différent de H; et,

(g) leurs sels d'addition.

ledit procédé étant caractérisé en ce que

A) on fait réagir une 1-phényl-1,2-alcane-dione de formule

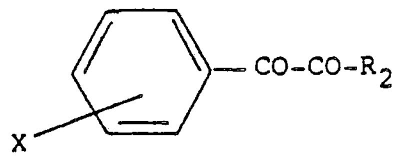

(II)

où X et $R_2$ sont définis comme ci-dessus, avec l'éthylène diamine

$$H_2NCH_2CH_2NH_2 \quad (III)$$

pour obtenir une 2-phényl-dihydropyrazine de formule

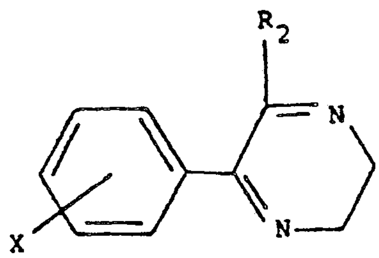

(IV)

où X et $R_2$ sont définis comme ci-dessus;

B) on soumet le composé de formule IV ainsi obtenu à une réaction de réduction au moyen d'un agent réducteur, notamment choisi parmi l'ensemble comprenant $LiAlH_4$ et $NaBH_4$ pour obtenir une 2-phényl-pipérazine de formule I où $R_1$ = $R_3$ = H; et

C) si nécessaire, on soumet le composé de formule I où $R_1$ = $R_3$ = H ainsi obtenu à une réaction d'alkylation pour introduire le groupe $R_1$ = alkyle en $C_1$—$C_4$ ou les groupes $R_1$ et $R_3$ représentant chacun un groupe alkyle en $C_1$—$C_4$.

**Patentansprüche für die Vertragsstaaten: BE CH DE FR GB IT LI LU NL SE**

1. Zur Familie der substituierten Phenyl-piperazine gehöriges Derivat, dadurch gekennzeichnet, daß es ausgewählt ist aus der folgenden Gruppe von Verbindungen:

1) 3-Methyl-2-phenylpiperazin,

2) 1-Isopropyl-3-phenylpiperazin,

3) 1-Ethyl-2-methyl-3-phenylpiperazin,

4) 1-Isopropyl-2-methyl-3-phenylpiperazin,

5) 1,2,4-Trimethyl-3-phenylpiperazin,

6) (Halogenophenyl)-alkylpiperazine, die in ihrem Molekül ein Halogenatom an dem Phenylring und mindestens eine Alkylgruppe an dem Piperazinylring aufweisen und der allgemeinen Formel

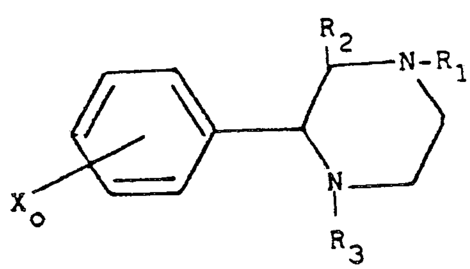

($I_o$)

entsprechen, worin $X_o$ F, Cl oder Br ist und $R_1$, $R_2$ und $R_3$ die oben angegebene Bedeutung haben, mit der zusätzlichen Bedingung, daß mindestens eines der genannten $R_1$, $R_2$ und $R_3$ von H verschieden ist, und

7) deren Additionssalze.

2. Derivat nach Anspruch 1, dadurch gekennzeichnet, daß es unter den (Halogenophenyl)-alkylpiperazinen ausgewählt ist, die der Formel

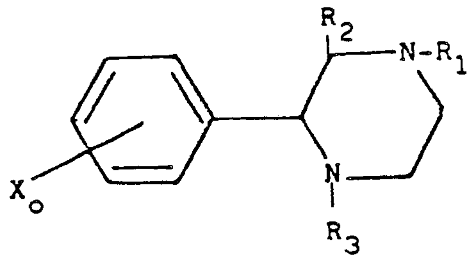

($I_0$)

entsprechen,

worin $X_0$ F, Cl oder Br ist und $R_1$, $R_2$ und $R_3$ die oben angegebene Bedeutung haben, wobei mindestens eines der genannten $R_1$, $R_2$ und $R_3$ von H verschieden ist,
und deren Additionssalze.

3. Derivat nach Anspruch 2, dadurch gekennzeichnet, daß $X_0$ 2-F, 2-Cl oder 2-Br ist.

4. 1-Ethyl-3-(2-fluorophenyl)-piperazin und dessen Additionssalze.

5. 3-Methyl-2-phenyl-piperazin und dessen Additionssalze.

6. 1-Ethyl-3-(2-chlorophenyl)-piperazin und dessen Additonssalze.

7. 1,2,4-Trimethyl-3-phenyl-piperazin und dessen Additionssalze.

8. Therapeutische Zusammensetzung, dadurch gekennzeichnet, daß sie in Verbindung mit einer physiologisch annehmbaren Grundmasse mindestens eine Phenylpiperazinderivat nach Anspruch 1 oder eines seiner nichttoxischen Additionssalze enthält.

9. Verwendung einer zu der Familie der Phenylpiperazinderivate nach Anspruch 1 und ihrer nichttoxischen Additionssalze gehörigen Substanz zur Darstellung eines antidepressiv wirkenden Medikaments, das zur therapeutischen Verwendung gegen Depressionen und depressive Zustände vorgesehen ist.

10. Verfahren zur Herstellung eines zu der Familie der Phenyl-piperazine gehörigen Derivats nach Anspruch 1 und ausgewählt aus der Gruppe, die durch die Verbindungen der Formel

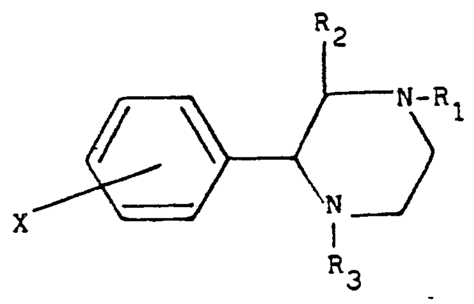

(I)

gebildet werden,
worin

(a) $X = R_1 = R_3 = H$ und $R_2 = CH_3$;

(b) $X = R_2 = R_3 = H$ und $R_1 = CH(CH_3)_2$;

(c) $X = R_3 = H$, $R_1 = CH_2CH_3$ und $R_2 = CH_3$;

(d) $X = R_3 = H$, $R_1 = CH(CH_3)_2$ und $R_2 = CH_3$;

(e) $X = H$ und $R_1 = R_2 = R_3 = CH_3$;

(f) X = F, Cl oder Br, $R_1$ ist H oder Alkyl mit $C_1$—$C_4$, $R_2$ ist H oder Alkyl mit $C_1$—$C_4$ und $R_3$ ist H oder Alkyl mit $C_1$—$C_4$, mit der zusätzlichen Bedingung für den Fall (f), daß mindestens eines der $R_1$, $R_2$ und $R_3$ von H verschieden ist; und

(g) deren Additionssalze,

dadurch gekennzeichnet, daß

A) ein 1-Phenyl-1,2-alkan-dion der Formel

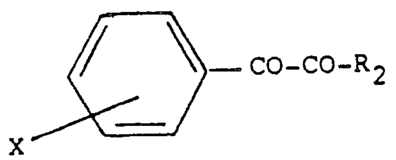

(II)

worin X und $R_2$ die oben angegebene Bedeutung haben, mit Ethylendiamin

$$H_2NCH_2CH_2NH_2 \qquad (III)$$

umgesetzt wird, um ein 2-Phenyl-dihydropyrazin der Formel

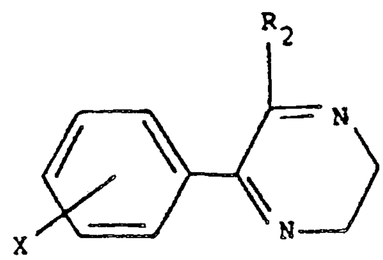

(IV)

zu erhalten,

worin X und $R_2$ die oben angegebene Bedeutung haben;

B) die so gewonnene Verbindung nach Formel IV einer Reduktionsreaktion mit Hilfe eines insbesondere aus der Gruppe $LiAlH_4$ und $NaBH_4$ gewählten Reduktionsmittels unterworfen wird, um ein 2-Phenyl-piperazin nach Formel I zu erhalten, worin $R_1 = R_3 = H$; und

C) nötigenfalls die so gewonnene Verbindung nach Formel I, worin $R_1 = R_3 = H$, einer Alkylierungsreaktion unterworfen wird, um die Gruppe $R_1$ = Alkyl mit $C_1$—$C_4$ oder die Gruppen $R_1$ und $R_3$, die jeweils eine Alkylgruppe mit $C_1$—$C_4$ darstellen, einzuführen.

**Patentanspruch für den Vertragsstaat: AT**

Verfahren zur Herstellung eines Derivats, das zur Familie der substituierten Phenyl-piperazine gehört und ausgewählt ist aus der Gruppe, die gebildet wird von den Verbindungen der Formel I

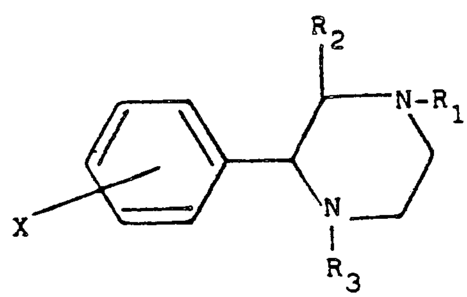

(I)

worin

(a) $X = R_1 = R_3 = H$ und $R_2 = CH_3$;
(b) $X = R_2 = R_3 = H$ und $R_1 = CH(CH_3)_2$;
(c) $X = R_3 = H$, $R_1 = CH_2CH_3$ und $R_2 = CH_3$;
(d) $X = R_3 = H$, $R_1 = CH(CH_3)_2$ und $R_2 = CH_3$;
(e) $X = H$ und $R_1 = R_2 = R_3 = CH_3$;
(f) X = F, Cl oder Br, $R_1$ ist H oder Alkyl mit $C_1$—$C_4$, $R_2$ ist H oder Alkyl mit $C_1$—$C_4$ und $R_3$ ist H oder Alkyl mit $C_1$—$C_4$, mit der zusätzlichen Bedingung für den Fall (f), daß mindestens eines der $R_1$, $R_2$ und $R_3$ von H verschieden ist; und
(g) deren Additionssalze,

welches Verfahren dadurch gekennzeichnet ist, daß

A) ein 1-Phenyl-1,2-alkan-dion der Formel

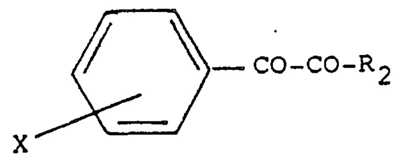

(II)

worin X und $R_2$ die oben angegebene Bedeutung haben, mit Ethylendiamin

$$H_2NCH_2CH_2NH_2 \quad (III)$$

umgesetzt wird, um ein 2-Phenyl-dihydropyrazin der Formel

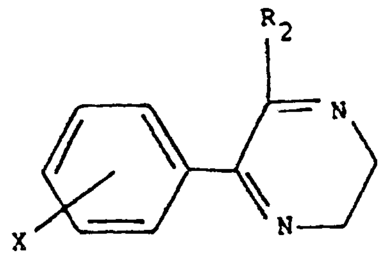

(IV)

zu erhalten,

worin X und $R_2$ die oben angegebene Bedeutung haben;

B) die so gewonnene Verbindung nach Formel IV einer Reduktionsreaktion mit Hilfe eines insbesondere aus der Gruppe $LiAlH_4$ und $NaBH_4$ gewählten Reduktionsmittels unterworfen wird, um ein 2-Phenyl-piperazin nach Formel I zu erhalten, worin $R_1 = R_3 = H$; und

C) nötigenfalls die so gewonnene Verbindung nach Formel I, worin $R_1 = R_3 = H$, einer Alkylierungsreaktion unterworfen wird, um die Gruppe $R_1$ = Alkyl mit $C_1$—$C_4$ oder die Gruppen $R_1$ und $R_3$, die jeweils eine Alkylgruppe mit $C_1$—$C_4$ darstellen, einzuführen.

**Claims for the Contracting States: BE CH DE FR GB IT LI LU NL SE**

1. A derivative belonging to the family of substituted phenylpiperazines selected from the group comprising:

1) 3-methyl-2-phenylpiperazine,
2) 1-isopropyl-3-phenylpiperazine,
3) 1-ethyl-2-methyl-3-phenylpiperazine,
4) 1-isopropyl-2-methyl-3-phenylpiperazine,
5) 1,2,4-trimethyl-3-phenylpiperazine,
6) (halogenophenyl)alkylpiperazines which have, in their molecule a halogen atom present on the phenyl ring and at least one alkyl group present on the piperazinyl ring, and which correspond to the general formula:

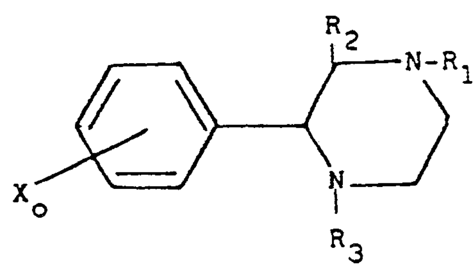

($I_o$)

wherein $X_o$ is F, Cl or Br and $R_1$, $R_2$ and $R_3$ are as defined above, with the additional proviso that at least one of the symbols $R_1$, $R_2$ and $R_3$ is different from H, and

7) addition salts thereof.

2. A derivative according to claim 1, which is selected from the group comprising the (halogenophenyl) alkyl-piperazines of the formula:

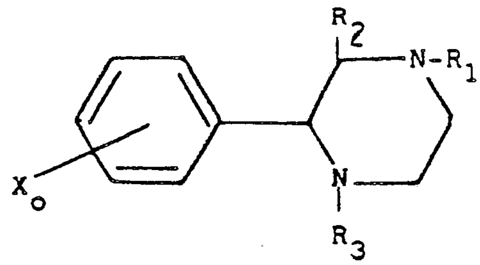

$(I_o)$

wherein $X_o$ is F, Cl or Br and $R_1$, $R_2$ and $R_3$ are as defined above, at least one of the symbols $R_1$, $R_2$ and $R_3$ being different from H; and additions salts thereof.

3. A derivative according to claim 2, wherein $X_o$ is 2-F, 2-Cl or 2-Br.

4. 1-Ethyl-3-(2-fluorophenyl)piperazine, and its addition salts.

5. 3-Methyl-2-phenylpiperazine, and its addition salts.

6. 1-Ethyl-3-(2-chlorophenyl)piperazine, and its addition salts.

7. 1,2,4-Trimethyl-3-phenylpiperazine, and its addition salts.

8. A therapeutic composition comprising, in association with a physiologically acceptable excipient, at least one phenylpiperazine derivative compound according to claim 1 or one of its non-toxic addition salts.

9. The use of a compound belonging to the family of phenylpiperazine derivatives according to claim 1 and their non-toxic addition salts, for the obtention of an antidepressant medicament destined to a therapeutical use vis-à-vis depressions and depressive states.

10. A method for the preparation of a compound belonging to the phenylpiperazine family according to claim 1 and selected from the group consisting of compounds of the formula

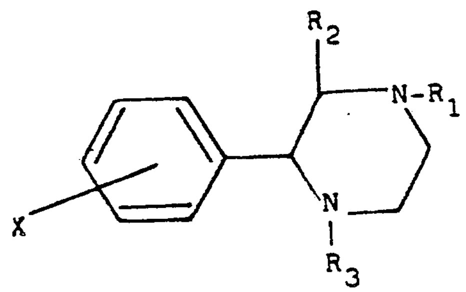

(I)

wherein:

(a) $X = R_1 = R_3 = H$, and $R_2 = CH_3$;
(b) $X = R_2 = R_3 = H$, and $R_1 = CH(CH_3)_2$;
(c) $X = R_3 = H$, $R_1 = CH_2CH_3$, and $R_2 = CH_3$;
(d) $X = R_3 = H$, $R_1 = CH(CH_3)_2$, and $R_2 = CH_3$;
(e) $X = H$, and $R_1 = R_2 = R_3 = CH_3$;
(f) X = F, Cl, or Br, $R_1$ is H or $C_1$—$C_4$ alkyl, $R_2$ is H, or $C_1$—$C_4$ alkyl, and $R_3$ is H or $C_1$—$C_4$ alkyl, with the additional proviso, with respect to said item (f), that at least one of the $R_1$, $R_2$ and $R_3$ is different from H; and
(g) addition salts thereof;

said method being characterized in that it comprises

A) reacting a 1-phenylalkane-1,2-dione of the formula:

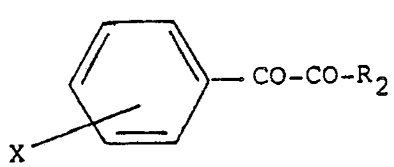

(II)

wherein X and $R_2$ are defined as above with ethylenediamine:

(III)

$$H_2NCH_2CH_2NH_2$$

to give a 2-phenyldihydropyrazine of the formula:

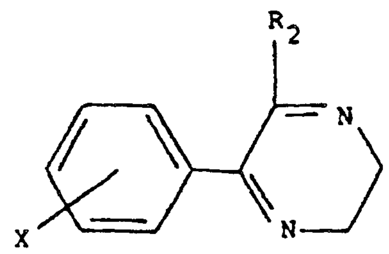

(IV)

wherein X and $R_2$ are defined as above;

B) subjecting the resulting compound of the formula IV to a reduction reaction with a reducing agent, selected in particular from the group comprising $LiAlH_4$, and $NaBH_4$, to give a 2-phenylpiperazine of the formula I, wherein $R_1 = R_3 = H$; and

C) if necessary, subjecting the resulting compound of the formula I wherein $R_1 = R_3 = H$ to an alkylation reaction in order to introduce the group $R_1 = C_1$—$C_4$ alkyl or the groups $R_1$ and $R_3$ each representing a $C_1$—$C_4$ alkyl group.

**Claim for the Contracting State: AT**

A method for the preparation of a compound belonging to the phenylpiperazine family and selected from the group consisting of compounds of the formula

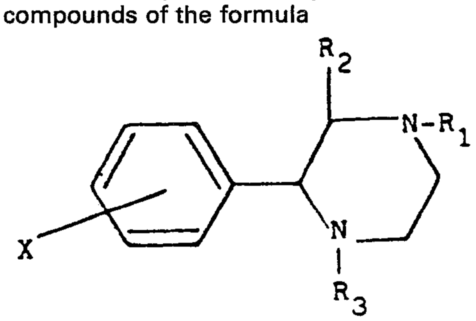

(I)

wherein:

(a) $X = R_1 = R_3 = H$, and $R_2 = CH_3$;

(b) $X = R_2 = R_3 = H$, and $R_1 = CH(CH_3)_2$;

(c) $X = R_3 = H$, $R_1 = CH_2CH_3$, and $R_2 = CH_3$;

(d) $X = R_3 = H$, $R_1 = CH(CH_3)_2$, and $R_2 = CH_3$;

(e) $X = H$, and $R_1 = R_2 = R_3 = CH_3$;

(f) X = F, Cl, or Br, $R_1$ is H or $C_1$—$C_4$ alkyl, $R_2$ is H, or $C_1$—$C_4$ alkyl, and $R_3$ is H or $C_1$—$C_4$ alkyl, with the additional proviso, with respect to said item (f), that at least one of the $R_1$, $R_2$ and $R_3$ is different from H; and

(g) addition salts thereof;

said method being characterized in that it comprises

A) reacting a 1-phenylalkane-1,2-dione of the formula:

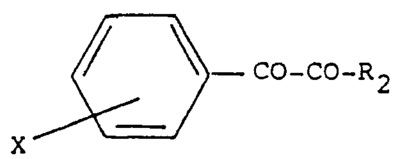

(II)

wherein X and $R_2$ are defined as above with ethylenediamine:

$$H_2NCH_2CH_2NH_2 \quad \text{(III)}$$

to give a 2-phenyldihydropyrazine of the formula:

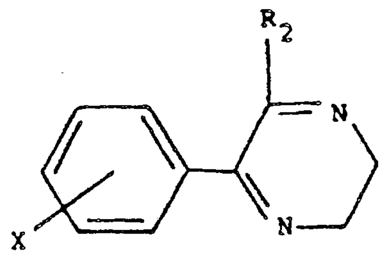

(IV)

wherein X and $R_2$ are defined as above;

B) subjecting the resulting compound of the formula IV to a reduction reaction with a reducing agent, selected in particular from the group comprising $LiAlH_4$, and $NaBH_4$, to give a 2-phenylpiperazine of the formula I, wherein $R_1 = R_3 = H$; and

C) if necessary, subjecting the resulting compound of the formula I wherein $R_1 = R_3 = H$ to an alkylation reaction in order to introduce the group $R_1 = C_1—C_4$ alkyl or the groups $R_1$ and $R_3$ each representing a $C_1—C_4$ alkyl group.